Europäisches Patentamt

(19) European Patent Office

Office européen des brevets

(11) Publication number: **0 183 460 B1**

# EUROPEAN PATENT SPECIFICATION

(45) Date of publication of patent specification: **16.10.91**   (51) Int. Cl.⁵: **A61F 13/15**

(21) Application number: **85308372.3**

(22) Date of filing: **18.11.85**

(54) **Disposable Diaper.**

(30) Priority: **16.11.84 NZ 210238**

(43) Date of publication of application:
**04.06.86 Bulletin 86/23**

(45) Publication of the grant of the patent:
**16.10.91 Bulletin 91/42**

(84) Designated Contracting States:
**AT BE CH DE FR GB IT LI LU NL SE**

(56) References cited:
**FR-A- 1 165 506**
**GB-A- 1 386 728**
**. US-A- 3 923 577**
**US-A- 4 519 798**

(73) Proprietor: **Trigon Packaging Systems (NZ) Limited**
**Corner Avalon Drive and Foreman Road**
**Hamilton(NZ)**

(72) Inventor: **Catcheside William Peter**
**56 Dinsdale Road**
**Hamilton(NZ)**

(74) Representative: **Gordon, Richard John Albert**
**R.J. Gordon & Company 17 Richmond Hill**
**GB-Richmond-upon-Thames, Surrey TW10 6RE(GB)**

Rank Xerox (UK) Business Services

## Description

This invention relates to disposable diapers.

A disposable diaper generally comprises a first sheet of liquid pervious non-woven textile material, a second sheet of liquid impervious polyethylene film superimposed on the first sheet and an absorbent pad of paper tissue about 8mm deep between the two sheets.

Such diapers are manufactured by depositing parallel tracks of hot melt adhesive about 10mm apart onto one face of a continuously fed web of polyethylene film about 300mm wide. Separate absorbent paper tissue pads of slightly smaller transverse dimension than the web are deposited successively in spaced relation on the web leaving a continuous border about 15mm wide around each pad. A second web of non-woven textile material then is superimposed on the pads and the polyethylene web such that the web of non-woven textile material adheres to the polyethylene web in the border by contact with the exposed tracks of hot melt adhesive. The combination is then severed to provide a plurality of diapers.

A disadvantage of such diapers is that the first and second sheets are secured one to another only where there is hot melt adhesive. In consequence, there are gaps between the tracks of adhesive and, in use, liquid contained in the pads tends to leak through these gaps.

Furthermore, the use of hot melt adhesive is unsatisfactory because it requires the provision of a bath of adhesive, a special applicator and a special conformation of feed rolls and heaters.

A more satisfactory method of manufacture would be to dispense with parallel tracks of hot melt adhesive and, instead, make use of a film of plastics material having two faces one of which is capable of being rendered adhesive by thermal activation whereby a surface of a liquid absorbent pad located between the film and a sheet of liquid pervious non-woven textile material contacts the adherent face of the film and adheres thereto by thermal activation of the film.

In FR-A-1 165 506 reference is made to activation by heating of a copolymer and the connection of two polymers without using any adhesive. However, the problem with substituting existing materials with such polymers in the manufacture of diapers is the necessity of providing pressure in addition to heat and the fact that the liquid absorbent pads are of significant thickness.

The invention is characterised in that the film and the sheet of liquid pervious non-woven textile material are nipped together in the border while the film is adherent.

In consequence, heat and pressure is applied to unite the film and sheet in the border without crushing the absorbent pad.

One embodiment of the invention is now described with reference to the accompanying drawings in which:

Figure 1:  is a diagram of a diaper partly cut away to show its structure, and

Figure 2:  is a diagram of the apparatus for its manufacture, with the nip rolls shown in section for clarity.

Figure 3:  is an end view of the nip rolls.

Figure 4:  is a diagram of a section of a fragment of film showing the different layers.

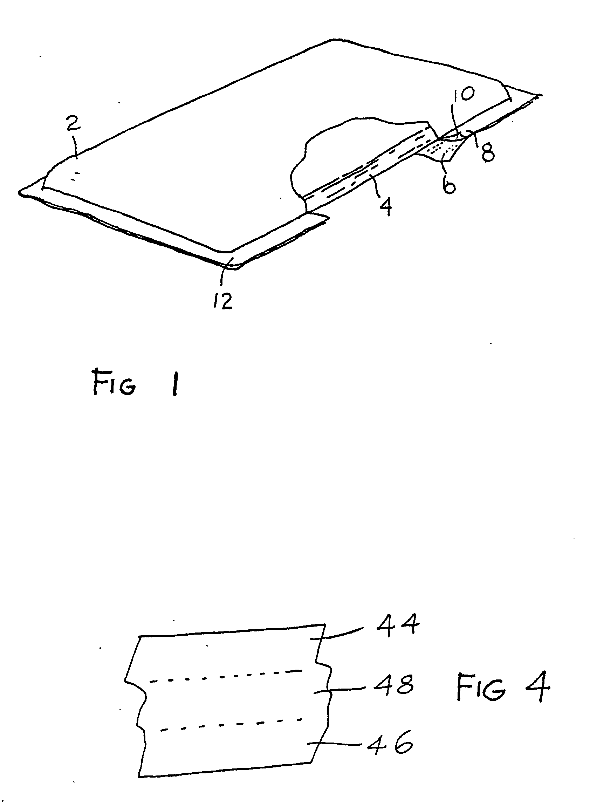

Referring firstly to figure 1, the diaper is of common three ply construction, the film ply 2, the pad 4 and the liner ply 6. The liner ply 6 a non-woven web of polyester fibres. The pad 4 of the paper tissue is standard inclusion of diapers but the film 2 is a triple layer HAC film 25 microns thick having a matt face 8 and a heat activated face 10. The heat activated face contacts the pad while the matt face lies outermost. The film 2 and liner ply define a continuous mutually adhered border 12 around the pad. The film is layered as shown In FIG 4 and the layers vary in the quantity of adhesion they develop when heated, depending on the vinyl acetate content. Those at the 30% end of the range are better for this purpose than those at the 10% end. The use of triple layer film allows one face to be rendered adherent leaving the opposite face non-adherent. The appearance and feel of the opposite face can be modified by the use of fillers while the centre layer supplements the film strength. A double layer film is therefore acceptable but not so suited to requirements.

Securing tapes (not shown) are affixed in the usual way.

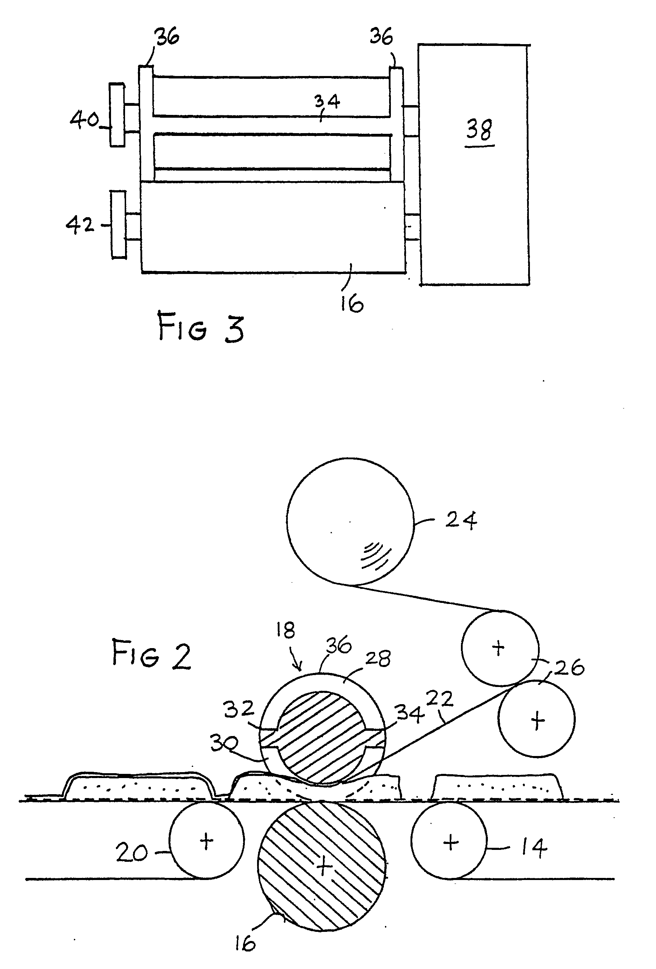

Referring now to figure 2, an endless conveyor 14 feeds a continuous band of non woven ply toward a pair of nip rolls 16, 18 both of which are induction-heated rolls.

Pads 4 are placed in correct alignment on the non woven ply leaving a border of about 12mm at the parallel edges of the band. A like conveyor 20 conducts the product away from the nip rolls. A band of film 22 unwinds from roll 24. A pair of driven feed rolls 26 feed the film toward nip roll 16, 18. The lower nip roll 16 is cylindrical whereas the upper roll has a pair of cavities 28, 30 about 4mm deep each equal in circumferential length to the length of a pad. The cavities are separated by a pair of bars 32, 34 which nip the longitudinal border areas leaving the transverse borders to be nipped by rims 36. Both rolls are heated to 100-120°C by induction from circuitry in housing 38 and driven by wheels 40, 42, the actual temperature depending upon the turning speed and the vinyl acetate content of the heat activated layer.

At speeds up to 200 units a minute these heat the film to about 100°C temporarily activating the adhesive layer. Temperatures of 100, 101, 102, 103, 104, 105, 106, 107, 108, 109, 110, 111, 112, 113, 114, 115, 116, 117, 118, 119, 120°C would therefore be suitable for some combinations of speed and acetate content. Likewise hot roll temperatures would depend on speed and thickness of the liner ply but would typically be 130, 135, 140, 145, 150, 155, 160, 165, 170, 175 or 180°C.

Referring now to FIG 4 the heat activatable layer 44 is made of ethylene vinyl acetate copolymer made of stock obtainable Du Pont under the Registered Trade Mark ELVAX. The opposite layer 46 is made of low density or linear polyethylene containing fillers to render it matt and soft to the touch. The central layer 48 is made of unmodified low density or linear low density polyethylene to supplement the strength of the three layers.

In another version the cavities are divided into sections by ribs creating a striated nip roll. This produces lines of adhesion about 10mm apart and 2mm across.

In another embodiment the liner ply is rayon.

## Claims

1. A method of manufacturing a diaper comprising laminating a liquid permeable liner (6) and a plastics film (2) of substantially the same area, the plastics film (2) having two opposite facing surfaces one (10) of which faces the liner (6) and is capable of being rendered adhesive by thermal activation while the other surface (8) of which is thermally stable, and there being located between the liner (6) and the film (2) a liquid absorbent pad (4) of smaller dimension than each of the liner (6) and the film (2) thereby providing a continuous border around the pad (4), such lamination being caused by heating the film (2) in the border and at least over part of the plastics film (2) which contacts the pad (4) to render the film (2) adherent characterised in that the method also includes the step of nipping the liner (6) and the film (2) together in the said border while the film (2) is adherent.

2. A method as claimed in claim 1 characterised in that the film (2) is heated to 100 - 120 degrees C.

3. A method as claimed in claim 1 or claim 2 characterised in that a surface which heats the plastics film ply (2) is heated to 150 - 180 degrees C.

4. A method as claimed in any one of the preceding claims characterised in that the film (2) is a triple layer ply.

5. A method as claimed in claim 4 characterised in that a heat activatable layer (44) of the ply is an ethylene vinyl acetate copolymer.

6. A method as claimed in claim 5 characterised in that a layer (46) remote from the heat activatable layer (44) of the ply is a low density polyethylene containing filler material.

7. A method as claimed in claim 6 characterised in that a layer (48) intermediate the outer layers (44,46) of the ply is an unmodified low density polyethylene.

8. A method as claimed in any one of claims 50 to 7 characterised in that the copolymer contains 10 - 30% vinyl acetate.

9. A method as claimed in any one of the preceding claims characterised in that the border is formed by passing the pad (4), the liner (6) and the film (2) between heat activating rollers (16,18) having means (32,34 and 36) which, when the rollers rotate, co-operate with one another to form the continuous border.

10. A diaper manufactured in accordance with any one of the preceding claims.

## Revendications

1. Procédé de fabrication d'une serviette hygiénique, dans lequel on assemble par laminage une couverture (6) perméable aux liquides et un film en matière plastique (2) ayant sensiblement la même étendue, le film en matière plastique (2) comportant deux surfaces opposées dont l'une (10) est en face de la couverture (6) et est capable d'être rendue adhésive par activation thermique et dont l'autre surface (8) est thermiquement stable, un coussinet (4) capable d'absorber des liquides étant placé entre la couverture (6) et le film (2) et ayant une plus petite taille que la couverture (6) et que le film (2), laissant ainsi une bordure continue autour du coussinet (4), ledit assemblage par laminage étant causé par chauffage du film (2) sur la bordure et sur au moins une partie du film (2) qui est en contact avec le coussinet (4) de façon à rendre le film (2) adhérent, caractérisé en ce que le procédé comporte aussi l'étape consistant à pincer ensemble la couverture (6) et le film (2) sur ladite bordure pendant que le film (2) est adhérent.

**2.** Procédé selon la revendication 1, caractérisé en ce que le film (2) est chauffé à 100 - 120 °C.

**3.** Procédé selon la revendication 1 ou 2, caractérisé en ce qu'une surface qui chauffe le pli de film en matière plastique (2) est chauffée à 150 - 180 °C.

**4.** Procédé selon l'une des revendications précédentes, caractérisé en ce que le film (2) est un pli à trois couches.

**5.** Procédé selon la revendication 4, caractérisé en ce qu'une couche (44) du pli, susceptible d'être activée thermiquement, est faite d'un copolymère d'éthylène et d'acétate de vinyle.

**6.** Procédé selon la revendication 5, caractérisé en ce qu'une couche (46) du pli, éloignée de la couche (44) susceptible d'être activée thermiquement, est faite d'un polyéthylène à basse densité contenant une charge.

**7.** Procédé selon la revendication 6 caractérisé en ce qu'une couche (48) située entre les couches extérieures (44, 46) du pli est faite d'un polyéthylène à basse densité non modifié.

**8.** Procédé selon les revendications 5 à 7, caractérisé en ce que le copolymère contient de 10 à 30 % d'acétate de vinyle.

**9.** Procédé selon l'une des revendications précédentes, caractérisé en ce qu'on forme la bordure en faisant passer le coussinet (4), la couverture (6) et le film (2) entre des rouleaux d'activation thermique (16, 18) comportant des moyens (32, 34 et 36) qui, lorsque les rouleaux tournent coopèrent pour former la bordure continue.

**10.** Serviette hygiénique fabriquée selon l'une des revendications précédentes.

**Patentansprüche**

**1.** Verfahren zur Herstellung einer Windel mit Laminieren einer flüssigkeitsdurchlässigen Einlage (6) und einer Kunststoffolie (2) von im wesentlichen gleicher Fläche, wobei die Kunststoffolie (2) zwei entgegengesetzte Oberflächen aufweist, wobei eine (10) der Einlage (6) gegenüberliegt und in der Lage ist, durch thermische Aktivierung haftend zu sein, während die andere Oberfläche (8) thermisch stabil ist, und wobei zwischen der Einlage (6) und der Folie (2) ein flüssigkeitsabsorbierendes Polster (4) von kleinerer Abmessung als die Einlage (6) und die Folie (2) angeordnet ist, wodurch eine kontinuierliche Einfassung um das Polster (4) geschaffen wird, wobei das Laminieren durch Erhitzen der Folie (2) in der Einfassung und mindestens über einen Teil der Kunststoffolie (2), die das Polster berührt, bewirkt wird, um die Folie (2) haftend zu machen, **dadurch gekennzeichnet,** daß das Verfahren den Schritt des Zusammenquetschens der Einlage (6) und der Folie (2) in der Einfassung enthält, während die Folie (2) haftend wird.

**2.** Verfahren nach Anspruch 1, **dadurch gekennzeichnet,** daß die Folie (2) auf 100 - 120 Grad Celsius erhitzt wird.

**3.** Verfahren nach Anspruch 1 oder 2, **dadurch gekennzeichnet,** daß eine Oberfläche, die die Kunststoffolienlage (2) erhitzt, auf 150 - 180 Grad Celsius erhitzt wird.

**4.** Verfahren nach einem der Ansprüche 1 - 3, **dadurch gekennzeichnet,** daß die Folie (2) eine dreilagige Schicht ist.

**5.** Verfahren nach Anspruch 4, **dadurch gekennzeichnet,** daß eine hitzeaktivierbare Schicht (44) der Einlage ein Ethylenvinylazetatcopolymer ist.

**6.** Verfahren nach Anspruch 5, **dadurch gekennzeichnet,** daß eine von der hitzeaktivierbaren Schicht (44) entfernt liegende Schicht (46) der Einlage ein Polyethylen niedriger Dichte mit einem Füllmaterial ist.

**7.** Verfahren nach Anspruch 6, **dadurch gekennzeichnet,** daß eine Schicht (48) zwischen den Außenschichten (44, 46) der Einlage ein ungeändertes Polyethylen niedriger Dichte ist.

**8.** Verfahren nach einem der Ansprüche 5 - 7, **dadurch gekennzeichnet,** daß das Copolymer 10 - 30 % Vinylazetat enthält.

**9.** Verfahren nach einem der Ansprüche 1 - 8, **dadurch gekennzeichnet,** daß die Einfassung durch Durchlaufen des Polsters (4), der Einlage (6) und der Folie (2) zwischen hitzeaktivierenden Walzen (16, 18) mit Einrichtungen (32,34,36) gebildet wird, die bei Drehung der Walzen miteinander zusammenwirken, um die kontinuierliche Einfassung zu bilden.

**10.** Windel, die nach einem der Ansprüche 1 - 9

hergestellt ist.

FIG 1

FIG 4

FIG 3

FIG 2